# EUROPEAN PATENT SPECIFICATION

(11) **EP 3 568 153 B1**
(45) Date of publication and mention of the grant of the patent: **23.09.2026**
(21) Application number: 18738758.4
(22) Date of filing: 12.01.2018
(51) Int. Cl.: A61K 38/48, A61P 1/18, A61P 7/00, A61P 11/00, A61P 31/04

(54) **BATROXOBIN FOR USE IN THE TREATMENT OF SEPSIS, ACUTE RESPIRATORY DISTRESS SYNDROME AND ACUTE PULMONARY DISORDER**
BATROXOBIN ZUR BEHANDLUNG VON SEPSIS, AKUTEM ATEMNOTSYNDROM UND AKUTEN LUNGENERKRANKUNGEN
BATROXOBINE DESTINÉE AU TRAITEMENT DE LA SEPTICÉMIE, DU SYNDROME DE DÉTRESSE RESPIRATOIRE AIGUË ET DES TROUBLES PULMONAIRES AIGUS

(30) Priority: 13.01.2017 JP 2017004219
(43) Date of publication of application: 20.11.2019
(73) Proprietor: Tobishi Pharmaceutical Co., Ltd., Tokyo 100-0006 (JP); Tokai University Educational System, Tokyo 151-8677 (JP)
(72) Inventor: MASUDA, Haruchika, Isehara-shi Kanagawa 259-1193 (JP); ASAHARA, Takayuki, Isehara-shi Kanagawa 259-1193 (JP); SENGA, Hirobumi, Tokyo 100-0006 (JP)
(74) Representative: Vossius & Partner Patentanwälte Rechtsanwälte mbB
(86) International application number: PCT/JP2018/001470
(87) International publication number: WO 2018/131724

(56) References cited:
- EP-A1- 3 075 726
- WO-A1-2007/037561
- WO-A1-2010/002035
- WO-A1-2010/086349
- WO-A1-2011/029931
- WO-A2-2004/069149
- DATABASE WPI Week 200522, Derwent World Patents Index; AN 2005-203083, XP002799959

## Description

### Technical Field

The present invention relates to a therapeutic agent for use in the treatment of specific diseases caused by neutrophil activation, the therapeutic agent containing batroxobin.

### Background Art

Blood includes erythrocytes, leukocytes, and platelets as cell components. Among them, leukocytes are immune competent cells involved in biological defense, and are classified into five types: neutrophils, eosinophils, basophils, lymphocytes, and monocytes. Among these, the number of neutrophil cells is the largest, and accounts for 50 to 70% of all the leukocyte types. Neutrophils have functions, for example, elimination of foreign substances such as bacteria and viruses which enter a living body from the outside.

When foreign substances such as bacteria enter a living body, macrophages immediately react therewith and release cytokines such as interleukin-1 (IL-1). Such cytokines cause inflammatory change in cells in the tissues. The tissues having the inflammatory change release many cytokines as represented by interleukin-8 (IL-8) and neutrophil migration stimulating factors.

Neutrophils, on the surface receptor, recognize the neutrophil migration stimulating factors and substances produced by bacteria by themselves, and activate the migration movement. After the migration, the neutrophils come into contact with, for example, bacteria, then recognize the bacteria as foreign substances via the surface receptor, and adhere and bind to the bacteria. The bound bacteria are engulfed by the plasma membrane of the neutrophils, incorporated into the neutrophils, and phagocytized.

The bacteria incorporated in the neutrophils are killed (phagocytized) by three means.

The first means for killing the bacteria is accomplished by reactive oxygen species such as hydrogen peroxide generated by the action of the enzyme system.

The second means for killing the bacteria is accomplished by bactericidal proteins and enzymes such as lysozyme and defensins released from granules in the neutrophils.

However, if reactive oxygen species or bactericidal proteins and enzymes are excessively released from the neutrophils, the tissue is damaged, and the inflammatory symptom further deteriorates.

The third means for killing the bacteria is accomplished by forming a chromatin web called NETs (neutrophil extracellular traps) through extracellular release of chromatin in the nucleus by activated neutrophils (Non Patent Literature 1). The cell death of the neutrophils that occurs in this process plays an important role in the action against bacteria. Since this is a different type of cell death from necrosis and apoptosis, it is called NETosis.

However, substances having antibacterial actions, such as histones, myeloperoxidase, and elastase, which are constituent components of NETs, are released into blood or tissue of a host, the substances also serve as damaging factors of the tissue and cells of the host.

Hence, it is believed that suppressing NETs formed by activated neutrophil can suppress an excessive inflammatory reaction.

From these, efforts have been made to suppress the inflammatory reaction by regulating neutrophil activation.

So far, several substances for regulating neutrophil activation have been reported.

For example, it has been reported that a histidine-rich glycoprotein, which is synthesized in a liver, contained in plasma, and known to be involved in regulation of a coagulation fibrinolysis system and control of angiogenesis, is a neutrophil activation regulator (Patent Literature 1).

Moreover, it has been reported that 2-adenosin N-pyrazole compounds and 2-adenosin thiophene compounds are useful as platelet aggregation inhibitors or neutrophil activation inhibitors (Patent Literatures 2 and 3).

Further, it has been reported that benzoxazinone derivatives and azetidinone derivatives are neutrophile infiltration suppressing agents and have anti-inflammatory actions (Patent Literatures 4 and 5).

Furthermore, a lactoferrin-containing inhibitor of formation of leukocyte extracellular traps and a lactoferrin-containing composition for treating a disease associated with formation of leukocyte extracellular traps have been reported (Patent Literature 6).
Patent Literature 7 relates to an activating agent of stem cells and/or progenitor cells comprising a thrombin-like enzyme which can be used in regenerative medicine.
Patent Literature 8 discloses a reptilase which is extracted from snake poison of pallas pit viper.
Patent Literature 9 relates to a therapeutic agent for a lower urinary tract disease such as cystitis, interstitial cystitis, prostatitis and benign prostatic hyperplasia.
Patent Literature 10 discloses certain thieno[2,3-C]pyridine derivatives, pharmaceutical compositions comprising the thieno[2,3-C]pyridine derivatives, and methods of use thereof. Patent Literature 11 relates to a composition comprising a modified interleukin 8 (IL-8), which is reported to have increased GAG binding affinity and further inhibited or downregulated GPCR activity compared to the respective wild type IL-8 for use in preventing or treating lung inflammation with neutrophilic infiltration.

### Citation List

### Patent Literature

Patent Literature 1: Japanese Patent No. 5807937
Patent Literature 2: Published Japanese Translation of PCT International Application No. 2003-506461
Patent Literature 3: Published Japanese Translation of PCT International Application No. 2003-502434
Patent Literature 4: Japanese Patent Application Publication No. Hei 5-148249
Patent Literature 5: Japanese Patent Application Publication No. Hei 7-242624
Patent Literature 6: International Publication No. WO2014/168253
Patent Literature 7: WO 2007/037561 A1
Patent Literature 8: CN 1 548 534 A
Patent Literature 9: WO 2010/002035 A1
Patent Literature 10: WO 2004/069149 A2
Patent Literature 11: WO 2011/029931 A1

### Non Patent Literature

Non Patent Literature 1: Brinkmann V et al: Neutrophil extracellular traps kill bacteria. Science. 303: 1532-1535, 2004

### Summary of Invention

### Technical Problem

However, from the viewpoints of efficacy, safety, and so forth, a novel neutrophil activation regulator and a therapeutic agent containing the regulator against diseases caused by neutrophil activation still have been demanded.

### Solution to Problem

The present inventors have conducted intensive studies to solve the above-described problems. As a result, the inventors have found that a specific trombin-like enzyme regulates neutrophil activation (particularly, degranulation, Mac-1 expression, NETs formation, transendothelial migration, and tissue infiltration) and is capable of treating diseases caused by neutrophil activation as defined in the appended claims. The present invention has been made based on such findings.

Specifically, the present invention relates to a therapeutic agent for use in the treatment of a disease caused by neutrophil activation, comprising batroxobin as the active ingredient, wherein the disease caused by neutrophil activation is selected from the group consisting of sepsis, acute respiratory distress syndrome, and acute pulmonary disorder.

The present invention also relates to the afore-said therapeutic agent for use in the treatment of the afore-mentioned disease(s) caused by neutrophil activation, wherein the neutrophil activation is regulated by inhibiting neutrophil NETs formation.

Herein disclosed are further the following [1] to [10].
[1] A neutrophil activation regulator comprising a thrombin-like enzyme as an active ingredient.
[2] The neutrophil activation regulator according to [1], wherein the thrombin-like enzyme is selected from the group consisting of batroxobin, ancrod, and crotalase.
[3] The neutrophil activation regulator according to [1], wherein the thrombin-like enzyme is batroxobin.
[4] The neutrophil activation regulator according to [1], wherein the neutrophil activation is regulated by inhibiting neutrophil degranulation.
[5] The neutrophil activation regulator according to [1], wherein the neutrophil activation is regulated by inhibiting neutrophil Mac-1 expression.
[6] The neutrophil activation regulator according to [1], wherein the neutrophil activation is regulated by inhibiting neutrophil NETs formation.
[7] The neutrophil activation regulator according to [1], wherein the neutrophil activation is regulated by inhibiting neutrophil transendothelial migration.
[8] The neutrophil activation regulator according to [1], wherein the neutrophil activation is regulated by inhibiting neutrophil tissue infiltration.
[9] A therapeutic agent against a disease caused by neutrophil activation, the therapeutic agent comprising the neutrophil activation regulator according to [1].
[10] The therapeutic agent according to [9], wherein the disease caused by neutrophil activation is selected from the group consisting of sepsis, acute respiratory distress syndrome, acute pancreatitis, and acute pulmonary disorder.

### Advantageous Effects of Invention

As described later in Examples, the neutrophil activation regulator containing a thrombin-like enzyme as an active ingredient regulates neutrophil activation (particularly, degranulation, Mac-1 expression, NETs formation, transendothelial migration, and tissue infiltration), and is utilizable as a therapeutic agent against diseases caused by neutrophil activation as defined in the appended claims.

### Brief Description of Drawings

Fig. 1 shows the result of verifying the inhibitory action of batroxobin on neutrophil degranulation elicited by TNF-α.
Fig. 2 shows the result of verifying the inhibitory action of batroxobin on neutrophil Mac-1 expression elicited by TNF-α.
Fig. 3 shows the result of verifying the inhibitory action of batroxobin on neutrophil NETs formation elicited by TNF-α.
Fig. 4 shows the result of verifying the inhibitory action of batroxobin on neutrophil transendothelial migration elicited by TNF-α.
Fig. 5 shows the result of verifying, by HE staining, the inhibitory action of batroxobin on neutrophil tissue infiltration into an ischemic hindlimb muscle tissue.
Fig. 6 shows the result of verifying, by MPO staining, the inhibitory action of batroxobin on neutrophil tissue infiltration into an ischemic hindlimb muscle tissue.

### Description of Embodiments

Hereinafter, the present invention will be described in detail.

It should be understood that the terms used in the specification are, unless otherwise specifically stated, used in the sense normally used in the art. Thus, unless otherwise defined, all the technical terms and scientific terms used in the specification have the same meaning as those generally understood by those skilled in the art to which the present invention pertains. If there is contradiction, the specification takes precedence including the definitions.

A neutrophil activation regulator containing a thrombin-like enzyme as an active ingredient is hereinafter also simply referred to as "regulator". The present invention relates to a therapeutic agent for use in the treatment of specific diseases caused by neutrophil activation, the therapeutic agent containing the regulator (hereinafter also simply referred to as "therapeutic agent").

An indicator of indicating "neutrophil activation" includes phenomena exhibited by neutrophils with stimuli of neutrophil activating factors, particularly, degranulation, Mac-1 expression, NETs formation, transendothelial migration, and tissue infiltration, and the like.

Neutrophil "degranulation" refers to a phenomenon in which a substance in granules is released outside the granules upon contact with a foreign substance or with a stimulus of a cytokine.

Neutrophil "Mac-1 expression" refers to a phenomenon in which a cell adhesion molecule (CD18/CD11b) is expressed on the neutrophil surface.

Neutrophil "NETs formation" refers to a phenomenon in which chromatin in the nucleus is extracellularly released to form a chromatin web.

Neutrophil "transendothelial migration" refers to a phenomenon in which neutrophils migrate apart from the blood circulation and enter a tissue through a gap of a vascular endothelial cell.

Neutrophil "tissue infiltration" refers to a phenomenon in which neutrophils slip through a vascular endothelial cell, and migrate and stay around a parenchymal cell of a tissue.

A "disease caused by neutrophil activation" refers to a disease that occurs by damage to a tissue or organ as a result of NETs formation, reactive oxygen species, and bactericidal proteins and enzymes excessively produced from neutrophils (activated neutrophils) indicating the above-described activation indicator. Specific examples of the disease include sepsis, acute respiratory distress syndrome,
acute pulmonary disorder, multiple organ failure, influenza-associated encephalopathy, epilepsy, viral encephalitis, and the like. Among these diseases, the present invention is suitably usable against sepsis, acute respiratory distress syndrome,
and acute pulmonary disorder. A further example of a disease caused by neutrophil activation disclosed herein is acute pancreatitis.

The "thrombin-like enzyme" used in the specification refers to a protease other than thrombin which has the characteristic of coagulating fibrinogen. Specific examples of the thrombin-like enzyme include batroxobin, ancrod, crotalase, flavoxobin, asperase, acutin, botropase, clotase, gabonase, venzyme, and the like. The thrombin-like enzyme used in accordance with the present invention is batroxobin.

The thrombin-like enzyme is classified into three categories based on a site in the substrate ,fibrinogen which the enzyme attacks. Specifically, the classified three categories are: (1) a protease (such as batroxobin, ancrod, crotalase) which separates only fibrinopeptide A from fibrinogen to generate fibrin I, (2) a protease (such as gabonase) which separates fibrinopeptide A and fibrinopeptide B from fibrinogen to generate fibrin II, which is also called fibrin, and (3) a protease (such as venzyme) which mainly separates fibrinopeptide B from fibrinogen.

In the specification, the term "fibrin I" refers to a monomer generated when only fibrinopeptide A separates from fibrinogen. This fibrin I is also called Des A fibrin.

Moreover, the term "fibrinopeptide A" refers to a peptide corresponding to the 16 amino acids at the NH₂ terminal end of the Aα chain of fibrinogen.

Moreover, the term "fibrinopeptide B" refers to a peptide corresponding to the 14 amino acids at the NH₂ terminal end of the Bβ chain of fibrinogen.

Moreover, in the specification, batroxobin, ancrod, crotalase, flavoxobin, asperase, acutin, and the like are mentioned as an example of the proteases which generate fibrin I from fibrinogen.

The thrombin-like enzyme used in accordance with the present invention is batroxobin. Ancrod, and crotalase are also described herein. All of these are known thrombin-like enzymes (Stocker KF: Snake venom proteins affecting hemostasis and fibrinolysis, in Medical Use of Snake Venom Proteins, Stocker KF, ed., CRC Press, Boston, p130-131; 1990).

Among batroxobin, ancrod (reference), and crotolase (reference), batroxobin is the most preferable.

Batroxobin is a thrombin-like enzyme derived from the venom of Bothrops moojeni, and is a glycoprotein having the molecular weight of approximately 36,000 Da. Batroxobin separates only fibrinopeptide A from fibrinogen and generates fibrin I (Aronson DL: Comparison of the actions of thrombin and the thrombin-like venom enzymes Ancrod and Batroxobin. Thrombos Haemostas (stuttg) 36: 9-13, 1976). Moreover, the primary structure of batroxobin has been already determined, and batroxobin is a single-chain glycoprotein composed of 231 amino acids (Itoh N et al: Molecular cloning and sequence analysis of cDNA for batroxobin, a thrombin-like snake venom enzyme. J Biol Chem 262: 3132-3135, 1987).

Batroxobin and thrombin are similar enzymes to each other in having a glycoprotein structure. Nevertheless, batroxobin separates only fibrinopeptide A from fibrinogen and generates fibrin I; meanwhile, thrombin differs from batroxobin in that thrombin separates not only fibrinopeptide A but also fibrinopeptide B from fibrinogen and generates fibrin II (also referred to as fibrin). Moreover, the two differ in that batroxobin does not act on blood coagulation factors other than fibrinogen, while thrombin acts on the other blood coagulation factors.

Batroxobin is a known substance, and can be prepared according to the method described in United States Patent No. 4137127. Additionally, batroxobin products are easily available from Tobishi Pharmaceutical Co., Ltd. (Tokyo, Japan) and Beijing Tobishi Pharmaceutical Co., Ltd. (China).

Ancrod is a thrombin-like enzyme derived from the venom of Agkistrodon rhodostoma, and is a glycoprotein having the molecular weight of approximately 35,400 Da. Ancrod, like batroxobin, separates only fibrinopeptide A from fibrinogen and generates fibrin I (Stocker KF: Snake venom proteins affecting hemostasis and fibrinolysis, in Medical Use of Snake Venom Proteins, Stocker KF, ed., CRC Press, Boston, p134-135; 1990).

Crotalase is a thrombin-like enzyme derived from the venom of Crotalus adamanteus, and is a glycoprotein having the molecular weight of approximately 32,700 Da. Crotalase, like batroxobin, separates only fibrinopeptide A from fibrinogen and generates fibrin I (Stocker KF: Snake venom proteins affecting hemostasis and fibrinolysis, in Medical Use of Snake Venom Proteins, Stocker KF, ed., CRC Press, Boston, p140-141; 1990).

The thrombin-like enzyme batroxobin used in the present invention can be a natural product or genetic recombinant product.

The regulator used in the present invention may be batroxobin alone, or may contain at least one additional thrombin-like enzyme.

Alternatively, the regulator of the present invention may contain the thrombin-like enzyme batroxobin in combination with at least one active substance (for example, a steroid, an immunosuppressant, or the like) other than the enzyme.

As the dosage form of the regulator of the present invention, dosage forms described in the Japanese Pharmacopoeia General Rules for Preparations can be used without particular limitation. Examples thereof include injections (including suspensions and emulsions) directly applied to a living body; external preparations such as ointments (including oleaginous ointments, emulsion ointments (creams), water soluble ointments, and the like), inhalants, liquids(including ophthalmic solutions, collunariums, and the like), suppositories, patches, cataplasms, and lotions; and internal preparations such as tablets (including sugar-, film-, gelatin-coated tablets), liquids, capsules, granules, powders (including fine granules), pills, syrups, and troches.

These preparations can be formulated by the method described in the Japanese Pharmacopoeia General Rules for Preparations.

Further, the regulator of the present disclosure may contain a pharmaceutically acceptable solid or liquid carrier or an interventional therapy base, depending on the dosage form. The pharmaceutically acceptable solid or liquid carrier includes a solvent, a stabilizer, a preservative, a solubilizing agent, an emulsifier, a suspending agent, a buffer agent, an isotonic agent, a coloring agent, a thickener, an excipient, a lubricant, a binding agent, a disintegraing agent, a coating agent, a corrigent, and the like.

The aforementioned descriptions of the dosage forms, the carriers, and the interventional therapy base are applied also to the therapeutic agent of the present invention.

The dosage and the number of administrations of the regulator of the present disclosure are normally changed depending on the type of the thrombin-like enzyme, the body weight of a patient, and the nature and state of a disease.

For example, in the case where batroxobin is administered as the thrombin-like enzyme to an adult once a day, the dosage is 0.1 to 50 Batroxobin Unit (abbreviated as BU). As a further preferable batroxobin dosage, the single dose to an adult is 1 to 20 BU, and the administration is performed every other day. In the case of external preparations, the dose is 0.01 to 500 mg per gram of an external preparation.

Herein, the batroxobin unit is a unit indicating the enzymatic activity of batroxobin; 2 BU is equivalent to the coagulation activity in 19.0 ± 0.2 seconds after 0.1 mL of a batroxobin solution is added to 0.3 mL of standard human citrated plasma at 37°C.

In the case where ancrod is administered as the thrombin-like enzyme to an adult human once a day, the dosage is 0.01 to 10 IU/kg, and a further preferable dosage is 0.5 IU/kg.

The regulator of the present disclosure can be administered to the subject by diluting the thrombin-like enzyme as appropriate, followed by: intravenous drip administration, intravenous injection, intraarterial injection, intramuscular injection, subcutaneous injection, intradermal injection, intracardiac injection, intraperitoneal injection, or subarachnoid injection; intrarectal administration, sublingual administration, nasal mucosa administration, transdermal administration, or inhalation; or topical administration into an organ and/or a tissue diseased by neutrophil activation. Preferably, the thrombin-like enzyme is diluted with 100 mL or more of saline and intravenously dripped for 1 hour or more.

The aforementioned descriptions of the dosages, the number of administrations, and the administration methods are applied also to the therapeutic agent of the present invention.

The acute toxicities (LD₅₀ (BU/kg)) of batroxobin on mouse, rat, rabbit, and dog are as shown in Table 1 below. The acute toxicity test has been evaluated by intravenous administration of batroxobin.

**Table 1: Acute Toxicity of Batroxobin (i.v.)**

| Animal Species | LD₅₀ value (BU/kg) |
|---|---|
| Mouse (ddy) | 192 ~ 210 |
| Rat (Wistar) | 105 ~ 110 |
| Rabbit (NW) | > 300 |
| Dog (mongrel) | 190 ~ 208 |

The regulator and the therapeutic agent of the present invention can be applied to animals having neutrophils. Specific examples of the animals include human, monkey, dog, pig, cat, rabbit, rat and mouse. Among these, human is preferable.

Hereinafter, while showing Preparation Example and Examples, the present invention will be specifically described. However, the present invention is defined in the appended claims and is not limited by the following Examples.

### [Preparation Example 1] Preparation of Regulator (Therapeutic Agent)

A batroxobin preparation having the following composition was formulated as an injection.

| Ingredient name | Blended amount |
|---|---|
| Batroxobin (active ingredient) | 10 BU |
| Chlorobutanol (preservative) | 3 mg |
| Gelatin hydrolysate (stabilizer) | 1 µL |
| Sodium chloride (isotonic agent) | 9 mg |
| Hydrochloric acid (pH regulator) | appropriate amount |
| Distilled water for injection | up to 1 mL |
| Total amount | 1 mL |

### [Preparation of Neutrophils]

### 1. Blood Collection

A healthy adult volunteer received an explanation about the experimental objective, and the consent was obtained. Then, 15 mL of peripheral venous blood was collected from the median cubital vein by using a 20-mL heparinized syringe.

### 2. Separation and Preparation of Neutrophils

As a blood-cell separation solution, a Polymorphprep density gradient solution (manufactured by PROGEN Biotechnik GmbH) was used. Onto 15 mL of the peripheral venous blood, an equal amount of the separation solution was overlaid, and centrifuged under a condition of 480 × g for 30 minutes. After mononuclear cells in the upper layer were removed by suction, polynuclear granulocytes in the lower layer were transferred to a 10-mL Hanks' buffer solution and centrifuged under a condition of 400 × g for 20 minutes. Then, the cell mass was suspended in 2 mL of BD Pharm Lyse^{™} (manufactured by Becton Dickinson Sciences) and subjected to lysis treatment in an ice bath for 5 minutes. After the lysis treatment, the cell suspension was centrifuged under a condition of 300 × g for 10 minutes. Subsequently, the cell mass was suspended again using a PBS-2mM EDTA buffer solution, and the final volume was adjusted to 15 mL. The resultant was used as human neutrophils in the following Examples.

### Example 1

### Inhibitory Action of Batroxobin on Neutrophil Degranulation Elicited by TNF-α

### 1. Experimental Method

Using 1% FBS-RPMI 1640 media, 1 × 10⁶ cells/100 µL of the neutrophil cell suspensions were prepared and placed in an ice bath until inoculation.

As a factor eliciting neutrophil degranulation, an inflammatory cytokine human recombinant TNF-α (hrTNF-α manufactured by Peprotech, Inc.) was used with a final concentration of 50 ng/mL.

To each 1% FBS-RPMI 1640 medium in a 24 well plate (manufactured by Greiner Bio One International GmbH) for suspension cells, batroxobin (DF-521 manufactured by Tobishi Pharmaceutical Co., Ltd.) (final concentration: 0.2 BU/mL) alone, human fibrinogen (hFbg manufactured by Sigma-Aldrich Co.) (final concentration: 2 mg/mL) alone, or a combination of batroxobin (final concentration: 0.2 BU/mL) with human fibrinogen (final concentration: 2 mg/mL) was added as test substances. Thus, conditioned medium (900 µL/well) was prepared.

As a positive control substance, N-formylmethionyl-leucyl-phenylalanine (fMLP manufactured by Sigma-Aldrich Co.) (final concentration: 20 nM) was used.

Note that, regarding the wells to which the human fibrinogen was added, the human fibrinogen was added in the end. After the human fibrinogen was added, the resultant was pre-incubated under a condition of 37°C for 15 minutes.

Regarding the wells to which the batroxobin and the human fibrinogen were added, when Des A fibrin gel formation was confirmed, 100 µL of the neutrophil suspension was inoculated into each conditioned medium (wells), and further cultured under a condition of 37°C for 60 minutes.

After the culturing was completed, the gel was removed using a 200-µL pipette. A PerCP-Cy5.5-labeled mouse anti-human CD66b antibody (manufactured by BioLegend, Inc.) was added to 400 µL of the conditioned medium containing the cultured neutrophils, and reacted with each other. Then, using a FACSVerse^{™} flow cytometry (manufactured by Becton Dickinson Sciences), CD66b-positive neutrophils were measured as degranulation neutrophils. The data were analyzed using FlowJo^{™} ver10.1 software (manufactured by Tommy Digital Biology Co., Ltd.), and the values were represented by mean fluorescence intensity (MFI).

### 2. Result

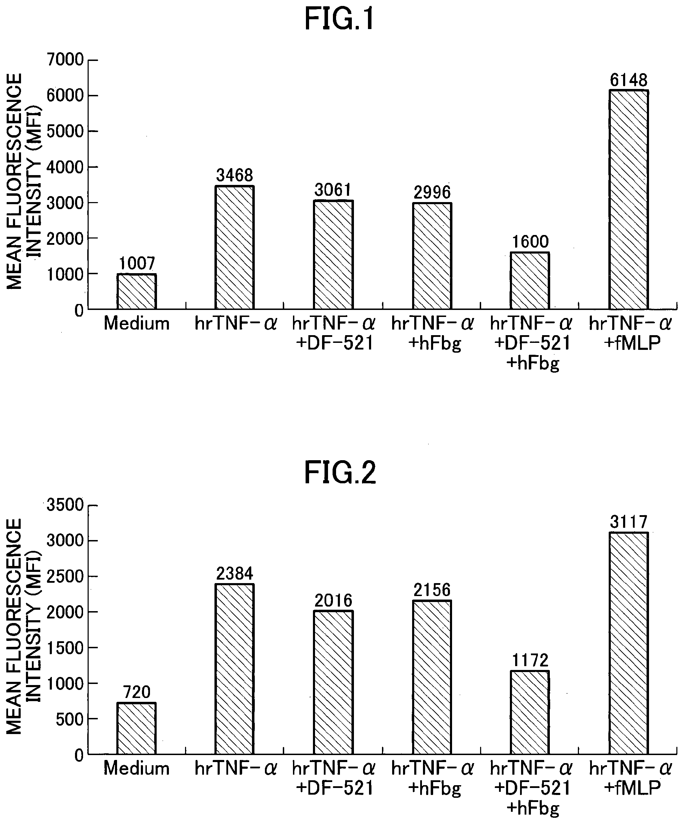

As shown in Fig. 1, the hrTNF-α elicited the neutrophil degranulation.

The fMLP as the neutrophil-activation positive substance clearly enhanced the neutrophil degranulation elicited by the hrTNF-α.

Adding the batroxobin (DF-521) alone and adding the human fibrinogen (hFbg) alone both had little influence on the neutrophil degranulation elicited by the hrTNF-α.

On the other hand, adding the batroxobin and the human fibrinogen clearly inhibited the neutrophil degranulation elicited by the hrTNF-α.

In this respect, fibrinogen is always present around neutrophils *in vivo.* Thus, batroxobin administered to a living body is capable of regulating neutrophil degranulation, that is, neutrophil activation, elicited by an inflammatory cytokine when inflammation occurs.

### Example 2

### Inhibitory Action of Batroxobin on Neutrophil Mac-1 (CD18/CD11b) Expression Elicited by TNF-α

### 1. Experimental Method

By the same method as in 1. Experimental Method of Example 1, neutrophils were co-cultured with each test substance.

After the culturing was completed, the gel was removed using a 200-µL pipette, an APC-Cy-labeled mouse anti-human CD11b antibody (manufactured by BioLegend, Inc.) and a PE-labeled mouse anti-CD18 antibody (manufactured by BioLegend, Inc.) were added into 400 µL of the conditioned medium containing the cultured neutrophils, and reacted with each other. Then, using a FACSVerse^{™} flow cytometry (manufactured by Becton Dickinson Sciences), Mac-1-positive neutrophils were measured as activated neutrophils. The data were analyzed using FlowJo^{™} ver10.1 software (manufactured by Tommy Digital Biology Co., Ltd.), and the values were represented by mean fluorescence intensity (MFI).

### 2. Result

As shown in Fig. 2, the hrTNF-α elicited the neutrophil Mac-1 expression.

The fMLP as the neutrophil-activation positive substance clearly enhanced the neutrophil Mac-1 expression elicited by the hrTNF-α.

Adding the batroxobin (DF-521) alone and adding the human fibrinogen (hFbg) alone both had little influence on the neutrophil Mac-1 expression elicited by the hrTNF-α.

On the other hand, adding the batroxobin and the human fibrinogen clearly inhibited the neutrophil Mac-1 expression elicited by the hrTNF-α.

In this respect, fibrinogen is always present around neutrophils *in vivo.* Thus, batroxobin administered to a living body is capable of regulating neutrophil Mac-1 expression, that is, neutrophil activation, elicited by an inflammatory cytokine when inflammation occurs.

### Example 3

### Inhibitory Action of Batroxobin on Neutrophil NETs formation Elicited by TNF-α

### 1. Experimental Method

A cover slip was put at the bottom of each well of a 24 well plate for suspension cells, and neutrophils were co-cultured with each test substance by the same method as in 1. Experimental Method of Example 1 described above.

Nevertheless, the final concentration of the positive control substance fMLP was set to 10 nM.

After the culturing was completed, the resultant was washed with PBS, and pre-fixed for 2 hours with a 0.1 M sodium phosphate buffer solution (pH7.4) containing 2.5% glutaraldehyde. After washing with a 0.1 M sodium phosphate buffer solution (pH7.4) for 10 minutes three times, 1% osmic acid was used for the fixation for 30 minutes.

Next, the resultant was dehydrated with 50%, 70%, 80%, and 90% ethanol for 10 minutes one time each, and further dehydrated with anhydrous ethanol for 10 minutes three times. The resultant was immersed in and substituted with t-butyl alcohol for 10 minutes three times, and freeze-dried (JFD-310 manufactured by JEOL Ltd.) with t-butyl alcohol.

The cover slips were taken out from the 24 well plate, and pasted on a sample stage of a scanning electron microscope by using an electro-conductive double-sided tape. A vapor was deposited using an osmium plasma coater (Neoc-Pro manufactured by Meiwafosis Co., Ltd.), and observed and imaged using a scanning electron microscope (JSM-6510LV manufactured by JEOL Ltd.) under an accelerating voltage condition of 15 kv.

### 2. Result

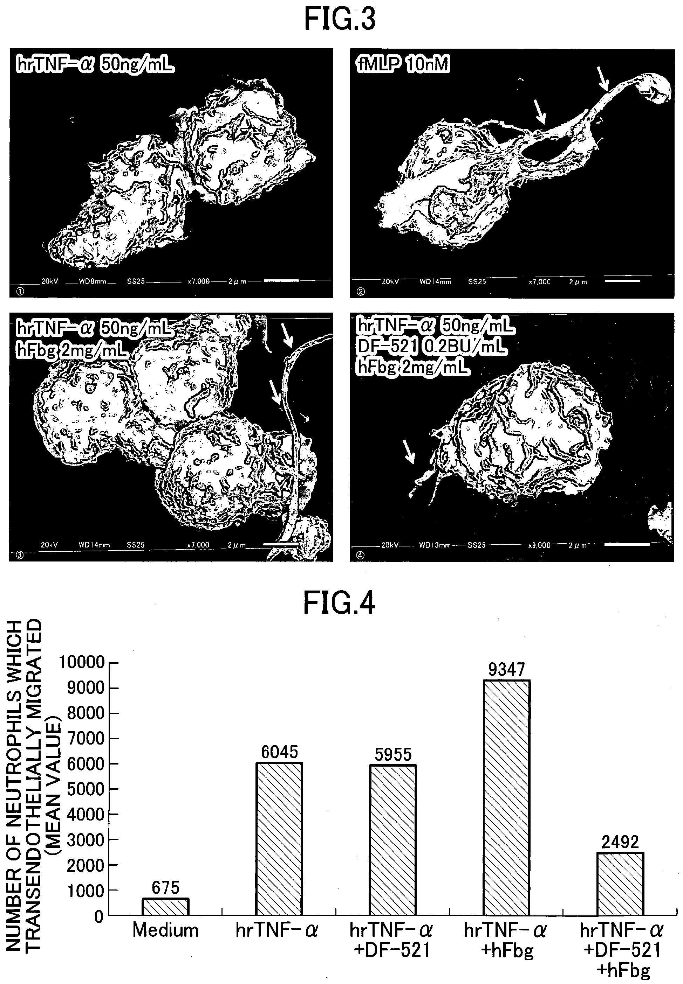

Fig. 3 shows the result (the arrows indicate NETs).

The hrTNF-α elicited the neutrophil NETs formation (the upper left in Fig. 3).

The fMLP as the neutrophil-activation positive control substance clearly caused the neutrophil NETs formation (the upper right in Fig. 3).

Adding the human fibrinogen alone clearly enhanced the neutrophil NETs formation elicited by the hrTNF-α (the lower left in Fig. 3).

On the other hand, adding the batroxobin and the human fibrinogen clearly inhibited the neutrophil NETs formation elicited by the hrTNF-α (the lower right in Fig. 3).

In this respect, fibrinogen is always present around neutrophils *in vivo.* Thus, batroxobin administered to a living body is capable of regulating neutrophil NETs formation, that is, neutrophil activation, elicited by an inflammatory cytokine when inflammation occurs.

### Example 4

### Inhibitory Action of Batroxobin on Neutrophil Transendothelial Migration Elicited by TNF-α

In this Example, the inhibitory action of batroxobin on neutrophil transendothelial migration was evaluated by employing an hrTNF-α-elicited neutrophil transendothelial migration assay.

### 1. Experimental Method

The neutrophil transendothelial migration assay was conducted according to the method of Pliyev et al. (Boris K. Pliyev et al, Molecular Immunology, 48, 1168-1177, 2011). As the endothelial cells, umbilical vein endothelial cells (Human umbilical vein endothelial cells, HUVECs, manufactured by Lonza Group) pre-cultured in a 5% FBS-EGM-2 endothelium growth medium (manufactured by Lonza Group) were used. A fibronectin-coated filter-equipped upper chamber (filter diameter: 6.5 mm, pore size: 3 µm, manufactured by Corning Incorporated) was inoculated with 200 µL of a cell suspension containing 7.0 × 10⁴ HUVECs having been re-adjusted with the 5% FBS-EGM-2 medium, while 800 µL of a 5% FBS-EGM-2 medium was added to a 24 well plate of a lower chamber. After culturing for 3 days, the filter of the upper chamber was filled with the HUVECs in a monolayer state.

On the day of the experiment, to the wells of the 24 well plate (manufactured by Greiner Bio One International GmbH) for culturing suspension cells, a cell suspension was added which had been prepared from the human neutrophils separated and prepared in [Preparation of Neutrophils] above such that the final cell concentration was 1.0 × 10⁷ cells/well in a 1% FBS-RPMI 1640 medium. Further, test substance solutions prepared in 1% FBS-RPMI 1640 media were added to the wells. The final concentrations of the test substances were: 0.2 BU/mL when batroxobin was added alone; 2.0 mg/mL when human fibrinogen was added alone; and 0.2 BU/mL for batroxobin and 2.0 mg/mL for human fibrinogen when the batroxobin and the human fibrinogen were added in combination. The neutrophils were pre-treated by culturing for 1 hour under conditions of these final concentrations and the final volume of the experiment systems being 1 mL.

The pre-treated neutrophils were collected and washed with PBS. Then, a suspension of the pre-treated neutrophils of 1.0 × 10⁷ cells/mL was prepared in a 1% FBS-RPMI 1640 medium.

Next, the upper chamber in which the HUVECs were alive was washed with 200 µL of 1% FBS-RPMI 1640 medium twice.
After the washing, to the upper chamber in which the HUVECs were alive, 100 µL of the suspension of the pre-treated neutrophils of 1.0 × 10⁷ cells/mL was added. To the lower chamber, an hrTNF-α solution prepared to a final concentration of 50 ng/mL in 1% FBS-RPMI 1640 medium was added and then cultured for 3 hours. The neutrophils were allowed to migrate to the lower chamber.

The human neutrophils which migrated to the lower chamber were collected, and the number was counted as transendothelial migration neutrophils by using a hemocytometer.

### 2. Result

As shown in Fig. 4, the hrTNF-α elicited the neutrophil transendothelial migration.

Adding the batroxobin (DF-521) alone had little influence on the neutrophil transendothelial migration elicited by the hrTNF-α.

Adding the human fibrinogen (hFbg) alone clearly enhanced the neutrophil transendothelial migration elicited by the hrTNF-α.

On the other hand, adding the batroxobin and the human fibrinogen clearly inhibited the neutrophil transendothelial migration elicited by the hrTNF-α.

In this respect, fibrinogen is always present around neutrophils *in vivo.* Thus, batroxobin administered to a living body is capable of regulating neutrophil transendothelial migration, that is, neutrophil activation, elicited by an inflammatory cytokine when inflammation occurs.

### Example 5

### Inhibitory Action of Batroxobin on Neutrophil Tissue Infiltration into Acute Hindlimb Ischemia Muscle Tissue

### 1. Experimental Method

### (1) Preparation of DIO mouse hindlimb ischemia models

In Charles River Laboratories Japan, Inc., male C57BL6/J mice at the age of 4 weeks after birth were continuously fed with high-fat diet (5.25 Kcal/g, D12492 manufactured by American Research Diet). Thereby, DIO (diet induced obesity) mice were prepared. The 10-week-old DIO mice were purchased from Charles River Laboratories Japan, Inc., fed with high-fat diet, and habituated for 2 weeks for use in the experiment.

Using the 12-week-old DIO mice, unilateral hindlimb ischemia models were prepared according to the method of Tsukada et al. (Tsukada S. et al: Identification of mouse colony-forming endothelial progenitor cells for postnatal neovascularization: a novel insight highlighted by new mouse colony-forming assay. Stem Cell Res Ther., 4 (1): 20-33, 2013). Specifically, the mice were each anesthetized by inhalation of 1.5 to 2.0% isoflurane (manufactured by Baxter Limited) to cut the skin at the distal end site of the inguinal ligament of the left hindlimb. After the ligation of the proximal end of the femoral artery, the distal end of the saphenous artery was ligated, and all the lateral branches were dissected and excised. Then, the skin opening was closed with a surgical stapler.

After the hindlimb ischemia models were prepared, saline was intraperitoneally administered to the model group (Model group), and 30 BU/kg of batroxobin was intraperitoneally administered to the batroxobin group (DF-521 group). Then, the mice were returned to the cages. The Sham Operation group was subjected to only the skin cutting.

On Day 1 (16 h) or Day 2 (36 h) after the hindlimb ischemia model preparation, Somnopentyl(registered trademark) diluted with 140 µL/mouse of saline at a ratio of 1:1 (64.8 mg/100 mL, manufactured by Kyoritsu Seiyaku Corporation) was intraperitoneally injected, and the whole blood was collected from the heart under anesthesia. The whole blood was lysed by using BD Pharm Lyse^{™} Lysing buffer (manufactured by BD Biosciences). After the lysis, the cells were stained with Ly6C-PE and Ly6G-PerCP Cy5.5 (manufactured by Biolegend, Inc.) by using a rabbit antimouse antibody. Monocytes and neutrophils in 1 mL of the blood were respectively evaluated as Ly6C⁺ Ly6G⁻ cell population and Ly6C⁺ Ly6G⁺ cell population.

### (2) Histological Examination

After the blood collection under anesthesia, ischemic hindlimbs of the mice were excised and fixed overnight with 4% paraformaldehyde. The fixed tissues were embedded with paraffin to prepare pathological slide specimens for: histological examination, myeloperoxidase (MPO) immunohistochemical staining, and hamatoxylin-eosin (HE) staining. Using Target Retrieval Solution, pH 9.0 (manufactured by DAKO) and a microwave oven, the MPO antigen of the deparaffinization specimen was reconstituted under a condition of 98°C for 15 minutes. For the MPO immunohistochemical staining, a rabbit anti-MPO antibody (manufactured by Abcam plc.) 100-fold diluted with PBS containing 10% normal goat serum/0.25% casein was used as a primary antibody. The slide specimens were reacted with the primary antibody at 4°C overnight, and then washed with PBS. The Peroxidase activity in the tissues was blocked using 3% H₂ O₂ -MeOH at room temperature for 10 minutes. Subsequently, an HRP (horse radish peroxidase)-labeled secondary antibody (Histofine(registered trademark) SimpleStain^{™} Mouse MAX PO manufactured by Nichirei Biosciences Inc.) was added to the slide specimens and reacted with each other at room temperature for 1 hour. The specimens were washed with PBS, reacted with DAB (3,3'-Diaminobenzidine tetrahydrochloride manufactured by DAKO), and colored to visualize the MPO-positive cells. Further, the specimens were washed with PBS, and the nuclei were stained with hamatoxylin. The stained specimens were sealed with Marinol. As for a negative control, 500-fold diluted rabbit IgG (manufactured by DAKO) was used as a primary antibody. Each specimen was observed under an optical microscope (DP73(registered trademark) manufactured by Olympus Corporation). The MPO-positive cells were evaluated using cellSense(registered trademark) (manufactured by Olympus Corporation) software.

### (3) Statistical analysis

All the data were represented by mean values or the mean values ± SD. In the *in vivo* hindlimb ischemia experiment, the Kruskal-Wallis test was employed to conduct the analysis of variance among the groups, and the groups were compared. The P < 0.05 was employed as the level of the statistical significant difference.

### 2. Result

### (1) Influence of Batroxobin on Number of Neutrophils in Blood

**Table 2. Influence of batroxobin on the number of leukocytes in blood in DIO mice hindlimb ischemia models mean value ± SD × 10⁵ /mL blood; n = 2)**

| | Day 1 (16 h) | | |
|---|---|---|---|
| | Total leukocytes | Neutrophils | Monocytes |
| Model group | 13.5±3.3 | 9.59±1.81 | 0.57±0.28 |
| Batroxobin group | 5.4±2.0 | 3.29±0.60 | 0.32±0.13 |
| | | | |

| | Day 2 (36 h) | | |
|---|---|---|---|
| | Total leukocytes | Neutrophils | Monocytes |
| Model group | 10.7±5.4 | 6.07±2.96 | 0.51±0.22 |
| Batroxobin group | 13.6±6.3 | 10.28±6.36 | 0.52±0.12 |

As shown in Table 2, the total number of leukocytes on Day 1 (16 h) after the hindlimb ischemia model preparation was 5.4 × 10⁵ /mL in the batroxobin group which was smaller than 13.5 × 10⁵ /mL in the model group. On Day 2 (36 h), the total number of leukocytes in the batroxobin group was returned to the level of the model group on Day 1.

Similarly, the number of neutrophils and the number of monocytes on Day 1 (16 h) after the hindlimb ischemia model preparation were smaller in the batroxobin group than in the model group. Specifically, the number of neutrophils was decreased from 9.59 × 10⁵ /mL to 3.29 × 10⁵ /mL, and the number of monocytes was decreased from 0.57 × 10⁵ /mL to 0.32 × 10⁵ /mL. On Day 2 (36 h), both the number of neutrophils and the number of monocytes in the batroxobin group were returned to the levels of the model group on Day 1.

### (2) Inhibitory Action of Batroxobin on Neutrophil Tissue Infiltration into Ischemic Hindlimb Muscle Tissue (HE Staining)

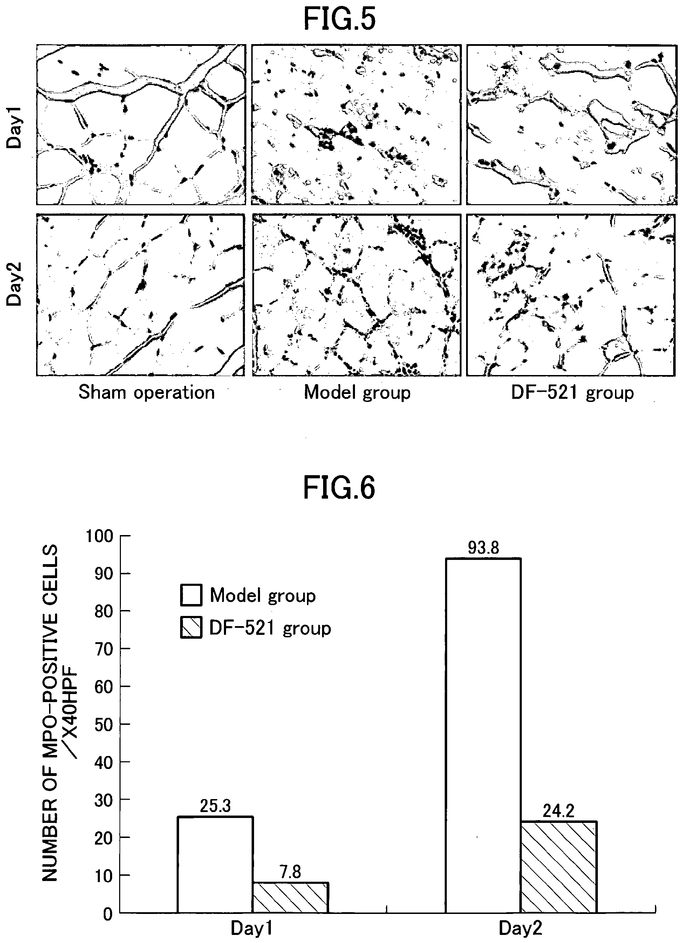

Fig. 5 shows images of the HE-stained ischemic hindlimb muscle tissues.

Regarding the model group (Model group), the neutrophil tissue infiltration into the ischemic hindlimb muscle tissue was greater on Day 2 than on Day 1 after the model preparation.

On the other hand, the neutrophil tissue infiltration of the batroxobin group (DF-521 group) was smaller than that of the model group. Particularly, on Day 2 after the model preparation, the neutrophil tissue infiltration of the batroxobin group was clearly smaller than that of the model group.

Thus, batroxobin administered to a living body is capable of regulating neutrophil tissue infiltration into an ischemic hindlimb muscle tissue, that is, neutrophil activation.

### (3) Inhibitory Action of Batroxobin on Neutrophil Tissue Infiltration into Ischemic Hindlimb Muscle Tissue (MPO staining)

The infiltration neutrophils observed as MPO immunohistochemical staining-positive cells were quantified using an optical microscope of ×40 high power field (HPF). Fig. 6 shows the result.

Regarding the model group (Model group), the neutrophil tissue infiltration on Day 2 after the model preparation was clearly greater than that on Day 1,and was 3.7 times (93.8/25.3) as great as that on Day 1.

On the other hand, regarding the batroxobin group (DF-521 group), the number of neutrophil tissue infiltrations was clearly smaller than that of the model group. On Day 1, the number was 30.8% of the model group; on Day 2, the number was 25.8% of the model group (P < 0.001).

Thus, batroxobin administered to a living body is capable of regulating neutrophil tissue infiltration into an ischemic hindlimb muscle tissue, that is, neutrophil activation.

### Industrial Applicability

The present invention is utilizable as a therapeutic agent against various diseases caused by neutrophil activation.

## Claims

1. A therapeutic agent for use in the treatment of a disease caused by neutrophil activation, comprising batroxobin as the active ingredient,
wherein the disease caused by neutrophil activation is selected from the group consisting of sepsis, acute respiratory distress syndrome, and acute pulmonary disorder.

2. The therapeutic agent for use according to claim 1, wherein the neutrophil activation is regulated by inhibiting neutrophil NETs formation.

## Patentansprüche

1. Ein Therapeutikum zur Verwendung bei der Behandlung einer durch Neutrophilen-Aktivierung verursachten Erkrankung, umfassend Batroxobin als Wirkstoff,
wobei die durch Neutrophilen-Aktivierung verursachte Erkrankung ausgewählt ist aus der Gruppe bestehend aus Sepsis, akutem Atemnotsyndrom und akuter Lungenerkrankung.

2. Das Therapeutikum zur Verwendung nach Anspruch 1, wobei die Neutrophilen-Aktivierung durch Hemmung der Bildung von Neutrophilen-NETs reguliert wird.

## Revendications

1. Agent thérapeutique pour une utilisation dans le traitement d'une maladie causée par une activation de neutrophiles, comprenant de la batroxobine en tant que principe actif,
dans lequel la maladie causée par une activation de neutrophiles est choisie dans le groupe constitué par une septicémie, un syndrome de détresse respiratoire aiguë, et une affection pulmonaire aiguë.

2. Agent thérapeutique pour une utilisation selon la revendication 1, dans lequel l'activation de neutrophiles est régulée par une inhibition de la formation de pièges extracellulaires de neutrophiles (NET).
